# EUROPÄISCHE PATENTSCHRIFT

(11) **EP 1 899 012 B1**
(45) Veröffentlichungstag und Bekanntmachung des Hinweises auf die Patenterteilung: **05.04.2017**
(21) Anmeldenummer: 06762211.8
(22) Anmeldetag: 27.06.2006
(51) Int. Cl.: A61Q 5/12, A61Q 7/00, A61P 17/14, A61K 8/44, A61K 8/97

(54) **MITTEL ENTHALTEND L-CARNITIN ODER L-CARNITINDERIVATE UND MINDESTENS EINE BESTIMMTE WEITERE SUBSTANZ**
AGENT CONTAINING L-CARNITINE OR L-CARNITINE DERIVATIVES AND AT LEAST ONE OTHER SPECIFIC SUBSTANCE
AGENTS CONTENANT DE LA L-CARNITINE, DES DERIVES DE L-CARNITINE ET AU MOINS UNE AUTRE SUBSTANCE

(30) Priorität: 05.07.2005 DE 102005031705
(43) Veröffentlichungstag der Anmeldung: 19.03.2008
(73) Patentinhaber: Henkel AG & Co. KGaA, 40589 Düsseldorf (DE)
(72) Erfinder: GIESEN, Melanie, 47608 Geldern (DE); SCHRÖDER, Klaus, Rudolf, 40822 Mettmann (DE); PETERSOHN, Dirk, 50996 Köln (DE)
(86) Internationale Anmeldenummer: PCT/EP2006/006193
(87) Internationale Veröffentlichungsnummer: WO 2007/003307

(56) Entgegenhaltungen:
- EP-A- 0 129 418
- WO-A-00/06188
- WO-A-02/36543
- WO-A-02/074265
- WO-A-2004/028495
- US-A- 6 080 788

## Beschreibung

Die vorliegende Erfindung betrifft Mittel, insbesondere zur Behandlung des Haares oder der Haut, enthaltend eine Kombination aus mindestens einer Verbindung ausgewählt aus a) L-Camitin, und/oder den Camitinderivaten Acetyl-L-Carnitin, L-Carnitin-Fumarat, L- Carnitin-Citrat, Lauroyl- L-Carnitin und insbesondere L-Carnitin-Tartrat sowie b) mindestens einer weiteren Substanz, welche ausgewählt ist aus Wirkstoffen ausgewählt aus Presssäften und Extrakten, die aus Echinacea purpurea gewonnen werden, und c) Taurin.

L-Carnitin ist ein vitaminähnlicher Wirkstoff, der mit den Vitaminen des B-Komplexes verwandt ist und essentiell wichtig für die Energieproduktion und den Fettstoffwechsel des menschlichen Körpers ist. Aus diesem Grund wird L-Carnitin vor allem als Nahrungsergänzungsmittel eingesetzt (US20040170709 A1). Ebenso wie L-Carnitin findet auch L-Carnitin-Tartrat Verwendung als Nahrungsergänzung, z.B. zur Unterstützung der Gewichtsreduktion (US20040028668 A1).

Carnitintartrat wird in der Offenlegungsschrift WO02/074265 A1 als Inhaltsstoff kosmetischer Mittel zur Behandlung von kosmetischen Mitteln zur Behandlung von Haaren offenbart. In der Offenlegungsschrift WO 2004/028495 A1werden Liposome enthaltend Carnitintartrat offenbart. Weiterhin offfenbart die WO 00/06188 A1 Zusammensetzungen zum Erhalt des Haarwuchses enthaltend immunregulatorische Inhaltsstoffe.

Die menschliche Haut mit Ihren Anhangsgebilden ist ein sehr komplex aufgebautes Organ, welches aus einer Vielzahl verschiedener Zelltypen besteht. Jede lebende Zelle dieses Organs ist in der Lage auf Signale ihrer inneren und äußeren Umwelt, zu reagieren. Diese Reaktionen der Zellen werden durch eine geordnete Regulation auf Gen- und Proteinebene realisiert, so dass der Metabolismus von Zellen der Haut und Ihrer Anhangsgebilde nicht statisch sondern sehr dynamisch ist. Die Reaktionen der Haut und/oder ihrer Anhangsgebilde auf Veränderungen der Umgebung dürfen jedoch nicht als Reaktionen einzelner, isolierter Zellen betrachtet werden. Vielmehr ist jede Zelle in ein komplexes Kommunikationsnetzwerk eingebunden. Dieses Netzwerk beinhaltet z.B. die Kommunikation zwischen Zellen der Epidermis und Zellen der Dermis. An der Kommunikation zwischen den Zellen der Haut und/oder ihrer Anhangsgebilde sind Signalmoleküle wie z.B. Interleukine, Wachstumsfaktoren (z.B. KGF, EGF oder FGF) usw. beteiligt.

Der Alterungsprozess ist ein grundlegender biologischer Prozess, der bei nahezu allen lebenden Organismen zu finden ist. Dementsprechend ist auch die menschliche Haut von diesem Phänomen betroffen. Die Hautalterung stellt sich als progressiver Vorgang dar, der zu einem Verlust der Hauthomöostase führt. Er wird von endogenen und exogenen Faktoren beeinflusst. Während die endogenen Aspekte als "genetisch gesteuertes Programm" ablaufen, sind für die exogenen Faktoren Umwelteinflüsse wie beispielsweise UV-Licht verantwortlich.

ATP (Adenosintriphosphat) ist die universelle Speicherform für chemische Energie in Zellen. Bei der Abspaltung der distalen Phosphatgruppe entsteht ADP und Pi (anorganisches Phosphat). Diese Reaktion ist stark exergon, d.h. es wird Energie frei. Produziert wird ATP beim zellulären, oxidativen Abbau von Fetten, Kohlehydraten und Proteinen. ATP dient der Zelle, auch den biologisch aktiven Zellen des Haarfollikels, als Energielieferant für biochemische Synthesen und Transportvorgänge. Diese Vorgänge sind endergon, d.h. sie laufen nur unter Energiezufuhr ab. Um ihren Stoffwechsel und zelluläre Strukturen optimal aufrecht zu erhalten und zu erneuern, sind Zellen also auf eine ausreichende Versorgung mit ATP angewiesen. So ist beispielsweise die Proliferation und Differenzierung von ORS-Keratinozyten (Keratinocyten der äußeren Wurzelscheide, "outer root sheath") an die ATP-Synthese gekoppelt, da für beide Vorgänge die Biosynthese spezifischer Proteine essentielle Voraussetzung ist. Auch dermale Papillenzellen benötigen beispielsweise zur Produktion von Wachstumsfaktoren und damit zur Steuerung des Haarzyklus ATP. Daher ist eine ausreichende Versorgung des Haarfollikels mit ATP essentielle Voraussetzung für kräftiges, vitales und gesundes Haar.

Die Menge eines haaraktiven Wirkstoffes, der üblicherweise transdermal und speziell transfollikulär bis zum Haarbulbus penetrieren kann, ist äußerst gering und hängt im Wesentlichen von den physikochemischen Eigenschaften der Substanz selber (z.B: Größe, Ladung, Lipophilie) sowie der Wahl der Formulierung ab.

Haarfollikelzellen unterliegen einem genetisch festgelegten Zyklus von Wachstum, Regression, und Ruhephase. Der Haarfollikel ist damit das einzige Organ, dass sich ständig selbst erneuert und somit einen, in Abhängigkeit von der jeweiligen Wachstumsphase, einzigartigen Metabolismus aufweist. So kommt der Metabolismus des Haarfollikels in der Ruhephase fast völlig zum Erliegen und wird mit jedem neuem Beginn eines weiteren Zyklus ebenfalls neu initiiert.

Gesteuert wird dieser Zyklus von einer kleinen, hochspezialisierten Zellpopulation im Haarbulbus, den dermalen Papillenzellen, die durch ein komplexes Set molekularer Signale, das spezifisch für jede Phase des Haarzyklus ist, das Haarwachstum kontrollieren (Botchkarev VA et al. (2003) J Investig Dermatol Symp Proc 8:46-55).

Die Gattung der Sonnenhutgewächse (Echinaceae) umfasst nordamerikanische Stauden, von denen einige schon seit langem zur unterstützenden Behandlung von Infekten (innerlich) u. Wunden (äußerlich) verwendet werden. Echinacea-Extrakte werden bekanntermaßen zur Stimulierung des Immunsystems und als antimikrobielle und antivirale Substanz eingesetzt Überraschenderweise wurde gefunden, dass durch die topische Anwendung von Mitteln enthaltend L-Carnitin oder genannte L-Carnitinderivate und mindestens einer weiteren Substanz, welche ausgewählt ist aus Wirkstoffen ausgewählt aus Presssäften und Extrakten, die aus Echinacea purpurea gewonnen werden, sowie Taurin, auf die behaarter Haut, insbesondere Kopfhaut, der Metabolismus dieser hochspezialisierten Zellen moduliert werden und die ATP-Synthese im Haarfollikel besonders stark stimuliert werden kann.

Weiterhin wurde überraschenderweise gefunden, dass die erfindungsgemäßen Mittel geeignet sind, dass die Freisetzung von Wachstumsfaktoren anzuregen und das Haar durch die Stimulierung der Proliferation der Haarkeratinozyten zu kräftigen und zu verdicken.

Die vorliegende Erfindung betrifft deshalb kosmetische und pharmazeutische Mittel, insbesondere zur Behandlung des Haares oder der Haut, enthaltend eine Kombination aus mindestens einer Verbindung ausgewählt aus a) L-Camitin, und/oder den Camitinderivaten Acetyl-L-Carnitin, L-Carnitin-Fumarat, L- Carnitin-Citrat, Lauroyl- L-Carnitin und insbesondere L-Carnitin-Tartrat sowie b) mindestens einer weiteren Substanz, welche ausgewählt ist aus Wirkstoffen ausgewählt aus Presssäften und Extrakten, die aus Echinacea purpurea gewonnen werden, und c) Taurin.

Die L-Carnitinderivate sind ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich.

Unter einem Wirkstoff, erhältlich aus Pflanzen der Gattung Echinacea, sind erfindungsgemäß Presssäfte und Extrakte von Echinacea purpurea (L) MOENCH zu verstehen.

Bevorzugt werden die Presssäfte bzw. Extrakte aus Kraut (den oberirdischen Pflanzenteilen) und/oder Wurzel der Sonnenhutgewächse gewonnen. Bevorzugter Weise werden die Presssäfte mit mechanischer Pressung gewonnen. Insbesondere bevorzugt ist eine Pressung nach dem von der Fa. Flachsmann patentierten Verfahren gemäß EP 0 730 830 B1.

Die Extrakte können mit Wasser, sowie polaren oder unpolaren organischen Lösungsmitteln sowie Mischungen davon in dem Fachmann bekannter Weise hergestellt werden. Extrakte, die durch Extraktion mit Ethanol oder Wasser/Ethanol-Mischungen, erhalten werden können, sowie Presssaft, sind bevorzugt.

Es können sowohl die Extrakte sowohl im ursprünglichen Extraktionsmittel als auch Extrakte/Presssaft in Wasser oder anderen organischen Lösungsmitteln und/oder deren Gemisch, insbesondere Ethanol sowie Ethanol/Wasser-Mischungen eingesetzt werden. Bevorzugt wird extrahiertes oder gepresstes Material als Feststoff eingesetzt, dem das Lösungsmittel (insbesondere möglichst schonend) entzogen wurde. Es können aber auch solche Extrakte/Presssäfte eingesetzt werden, denen das Lösungsmittel zum Teil entzogen wurde, so dass ein verdickter Extrakt/Presssaft eingesetzt wird. Ganz besonders bevorzugt sind Presssäfte aus dem frischen Echinacea purpurea Kraut (Echinacea purpurea Moench herba). Insbesondere werden die Extrakte und/oder Presssäfte in fester Form eingesetzt.

Es konnte gezeigt werden, dass die ATP-Menge in den behandelten Follikeln signifikant im Vergleich zur einer Placeboformulierung erhöht wird. Damit wird dem biologisch aktiven Teil des Haares signifikant mehr ATP als Energielieferant für biochemische Synthesen und Transportvorgänge zur Verfügung gestellt, so dass Stoffwechselvorgänge und zelluläre Strukturen optimal aufrecht erhalten und erneuert werden können. Das Haar wird dadurch gekräftigt und vitalisiert und kann Schädigungen besser reparieren bzw. neues Haar aufbauen. Die Steigerung der Stoffwechselaktivität begünstigt das Haarwachstum, da für die zu Grunde liegenden Prozesse ausreichend Bausteine wie z.B. Aminosäuren zum Proteinaufbau bereitgestellt werden müssen; die dafür benötigte Energie wird z.B. durch die Verstoffwechselung von Glucose bereitgestellt. Weiterhin wurde gefunden, dass der Einsatz der erfindungsgemäßen Mittel zu einer Anregung des Haarwachstums und einer Stärkung des vitalen Haares führen. Es konnte eine synergistischen Erhöhung der Expression der Wachstumsfaktoren HGF (Hepatozytenwachtumsfaktor, Hepatocyte Growth Factor) und KGF (Keratinozytenwachtumsfaktor, Keratinocyte Growth Factor) in dermalen Papillenzellen nachgewiesen werden. Die Wirkung eines Mittels enthaltend L-Carnithintartrat, Taurin und Presssaft aus Echinacea purpurea herba liegt um ca. 13% höher als die, die durch eine Addition der Einzeleffekte erreicht werden kann (vgl. Beispiel 21).

Ein besonderer Einfluss der erfindungsgemäßen Mittel konnte auf die biologische Haarverdickung festgestellt werden. Die Anregung der Keratinozyten der äußeren Wurzelscheide, die für die Bildung des Haarschaftes mitverantwortlich sind, erfolgt über die Wachstumsfaktoren HGF und KGF. Durch die Haarverdickung auf biologischer Basis werden Effekte wie die "Überpflege" der Haare vermieden. Das Haar wächst von der Wurzel an gestärkt und mit einem größeren Durchmesser nach, so dass dieser Effekt besonders langanhaltend ist. Dieser Effekt konnte in vivo ebenfalls nachgewiesen werden (vgl. auch Beispiel 26).

Ein weiterer Vorteil der vorliegenden Erfindung ist, dass die erfindungsgemäßen Mittel in der Lage sind, die Haarstruktur positiv zu beeinflussen, in dem die speziellen Haarspezifischen Strukturproteine (die Haarkeratine) stimuliert werden. So konnte überraschenderweise gezeigt werden, dass die Genexpression der Haarkeratine, hHa3-I und hHa4 signifikant erhöht wird. Dadurch wird die Haarstruktur, und somit das Haar, gekräftigt und gestärkt. Durch die Beeinflussung der Haarstruktur schon in der Haarwurzel kann das Haar kräftig und gesund nachwachsen, ohne Nebenerscheinungen wie z.B. die Akkumulation von Pflegesubstanzen auf der Haarfaser.

Weiterhin wurde gefunden, dass durch die Applikation der erfindungsgemäßen Mittel das Haar in seiner Struktur, seinem Wachstum und seinem Stoffwechsel positiv beeinflusst werden. Die Genexpression der dafür wichtigen Haargene wurde durch die Verwendung des erfindungsgemäßen Mittels signifikant reguliert. Insbesondere konnten erhöhte Expressionen von Genen beobachtet werden, die in der extrazellulären Matrix wichtig für die Verankerung des Haares in der Kopfhaut sind. Sowohl die dermale Papille, sowie der gesamte Bulbus ist mit extrazellulärer Matrix umgeben, die den Haarfollikel in der Kopfhaut verankert. Die Förderung der Synthese von extrazellulären Matrixproteinen wie Laminin und Collagen führt zu einer besonders guten Verankerung des Haares in der Kopfhaut.

Die Penetration von Wirkstoffen zum Follikel ist üblicherweise erschwert, da das entsprechende Target, die dermale Papille sowie die ORS-Keratinozyten, ca. 2 mm tief in der Kopfhaut eingebettet ist. Die Verwendung von Liposomen erhöht die Penetration eines Wirkstoffes, so dass liposomal verkapselte erfindungsgemäße Zusammensetzungen sehr gut wirken. Überraschenderweise wurde festgestellt, dass die erfindungsgemäßen Zusammensetzungen selbst dann eine ausreichende Penetration zum Wirkungsort zeigen, wenn die Verwendung von Liposomen aus formulierungstechnischen Gründen nicht möglich ist.

Die erfindungsgemäßen Mittel, insbesondere zur Behandlung des Haares oder der Haut, können L-Carnitin und/oder ein L-Carnitinderivat oder auch mehrere voneinander verschiedene L-Carnitinderivate enthalten.

L-Carnitin bzw. L-Carnitinderivate sind in den erfindungsgemäßen Mitteln bevorzugt in Mengen von 0,001 bis 10 Gew.-%, bezogen auf die gesamte Zubereitung, enthalten. Mengen von 0,1 bis 10 Gew.-% sind besonders bevorzugt, Mengen von 0,1 bis 5 Gew.-% sind in besonderem Maße bevorzugt, und Mengen von 1 bis 3 Gew.-% sind ganz besonders bevorzugt.

Taurin und/oder dessen Derivate sind in den erfindungsgemäßen Mitteln bevorzugt in Mengen von 0,001 bis 10 Gew.-%, bezogen auf die gesamte Zubereitung, enthalten. Mengen von 0,05 bis 5 Gew.-% sind besonders bevorzugt, Mengen von 0,1 bis 5 Gew.-% sind in besonderem Maße bevorzugt, und Mengen von 0,5 bis 3 Gew.-% sind ganz besonders bevorzugt.

Die Presssäfte und/oder Extrakte aus Echinacea sind in den erfindungsgemäßen Mitteln bevorzugt in Mengen von 0,001 bis 10 Gew.-%, bezogen auf die gesamte Zubereitung, enthalten. Mengen von 0,01 bis 5 Gew.-% sind besonders bevorzugt, Mengen von 0,01 bis 5 Gew.-% sind in besonderem Maße bevorzugt, und Mengen von 0,01 bis 2 Gew.-% sind ganz besonders bevorzugt.

Gemäß einer weiteren bevorzugten Ausführungsform der vorliegenden Erfindung kann das erfindungsgemäße Mittel auch Wirkstoffe, erhältlich aus Pflanzen der Familie Apiaceae, insbesondere der Gattung Foeniculum, insbesondere Foeniculum vulgare (Fenchel), enthalten. Es kann sich dabei insbesondere um Extrakte oder Presssäfte aus Pflanzenteilen oder dem Samen handeln, die z.B. in analoger Weise zu den vorstehend angegebenen Extraktionsmethoden für Echinaceae erhalten werden können. Bevorzugt ist insbesondere Fenchelsamenextrakt, beispielsweise hergestellt von der Fa. Flachsmann.

Insbesondere bevorzugt sind erfindungsgemäße Mittel enthaltend Fenchelsamenextrakt, L-Carnitin und/oder L-Carnitinderivate, bevorzugt L-Carnitintartrat, sowie ein Extrakt aus Echinacea, bevorzugt Presssaft aus Echinacea purpurea.

Die erfindungsgemäßen Mittel können zusätzlich Proteinhydrolysate umfassen, vorzugsweise kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Weiterhin können zusätzlich filmbildende Substanzen in die Formulierungen eingearbeitet werden, die auf das Haar aufziehen und es somit direkt spürbar verdicken. Dies kann vorteilhafterweise zusätzlich zum erfindungsgemäßen Effekt der Haarverdickung, welche auf biologischen Weg durch die Stimulierung des Haarfollikel erfolgt geschehen, so dass zusätzlich zu der erfindungsgemäßen längerfristigen Verdickung des Haares durch die Stimulierung des Haarfollikels eine kurzfristig fühlbare Wirkung der erfindungsgemäßen Mittel erzielt wird. Geeignete Filmbildner sind dem Fachmann bekannt und beispielsweise ausgewählt aus Polymeren, z.B. Polyvinylalkohol oder Polyvinylpyrrolidon sowie deren Copolymeren.

Hinsichtlich des zeitlichen Ablaufs unterliegt der Einsatz der erfindungsgemäßen Mittel keinerlei Beschränkungen. Es ist prinzipiell möglich, zwei separate Zubereitungen, enthaltend (a) L-Carnitin oder ein L-Carnitinderivat und (b) mindestens eine weitere Komponente, auch die erfindungsgemäßen Komponenten, Taurin und/oder dessen Derivate, und/oder ein Wirkstoff, erhältlich aus Pflanzen der Gattung Echinacea, nacheinander in beliebiger Reihenfolge auf die Haare aufzubringen. Hierbei sollte allerdings zwischen den Schritten (a) und (b) kein allzu großer zeitlicher Abstand liegen, so dass die Haare zwischen den Schritten nicht trocknen.

Die erfindungsgemäßen Mittel können entweder auf dem Haar verbleiben oder sie werden vorzugsweise nach einer Einwirkzeit von 10 Sekunden bis 60 Stunden ausgespült. Dieses Ausspülen kann mit reinem Wasser oder einem marktüblichen Shampoo erfolgen. Einwirkzeiten von 1 bis 15 Minuten haben sich in den meisten Fällen als ausreichend erwiesen.

Unabhängig von dem genauen Ablauf der Behandlung hat es sich als vorteilhaft erwiesen, die erfindungsgemäßen Mittel bei einer Temperatur von 20 bis 55 °C, insbesondere von 35 bis 40°C, anzuwenden.

Hinsichtlich der Art, gemäß die erfindungsgemäßen Mittel auf das Haar aufgebracht werden, bestehen keine prinzipiellen Einschränkungen.

Erfindungsgemäß von besonderem Interesse sind Haartonics, insbesondere als auf dem Haar verbleibende (leave on) Formulierung. Diese werden vorzugsweise bei Raumtemperatur angewendet, der alkoholische Gehalt liegt bevorzugtermaßen im Bereich von etwa 30 % bis etwa 35 % und der pH-Wert sollte etwa bei pH 7 liegen. Insbesondere bei Haartonics hat sich der Einsatz von in Liposomen verkapseltem L-Carnitin bzw. L-Carnitinderivaten als vorteilhaft erwiesen.

Es ist dabei möglich, sowohl die erfindungsgemäßen Komponenten zuerst zu mischen und dann die Mischung zu verkapseln. Es ist aber auch möglich, die jeweiligen Komponenten einzeln zu verkapseln und dann in der entsprechenden Menge in dem erfindungsgemäßen Mittel einzusetzen.

Die Mischung der Komponenten mit anschließender Verkapselung in Liposomen ist besonders bevorzugt. Solche Liposomen können insbesondere in Haartonics eingesetzt werden.

Wenngleich die zuvor genannten Haarbehandlungsmittel erfindungsgemäß bevorzugt sind, können die erfindungsgemäßen Kombinationen auch anderen Haarbehandlungsmitteln, wie z.B. Haarfärbemitteln und Wellmitteln, zugefügt werden. Diese Mittel enthalten dann gegebenenfalls die bekannten direktziehenden Farbstoffe, Vorläufer für Oxidationsfarbstoffe (Entwickler- und Kupplerkomponenten) und Oxidationsmittel bzw. Reduktionsmittel.

Vorteilhafterweise können die erfindungsgemäßen Mittel so das Haar vor der Beanspruchung bei der Färbung schützen, das Haarfollikel aktivieren und gleichzeitig Hautirritationen durch die Well- bzw. Haarfärbemitteln vermindern bzw. lindern.

Die erfindungsgemäßen Mittel sind ebenfalls für die Behandlung von Haut geeignet. Unter Haut im erfindungsgemäßen Sinne sind insbesondere menschliche Haut und Schleimhaut zu verstehen.

Die erfindungsgemäßen Mittel bewirken ebenfalls die Verdickung epithelialer Zellen und Zellschichten, insbesondere auf der Haut, eine Verbesserung der Festigkeit der Haut, die Stärkung der Epidermis, eine Verminderung des dünner Werdens der Haut, insbesondere durch Alterserscheinungen der Haut, eine Reduktion des transepidermalen Wasserverlustes der Haut, eine Verbesserung der Hautfeuchte, den Schutz der Haut vor Infektionen, exogenen Faktoren wie Smog, Zigarettenrauch sowie gegen die Beanspruchung durch schädigende und/oder reizende Stoff, insbesondere Tenside und/oder häufigen Wasserkontakt.

Als Konfektionierung der die erfindungsgemäßen Mittel enthaltenden Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole geeignet. Diese werden in der Regel auf wässriger oder wässrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wässrig alkoholischen Basis in einem Gewichtsverhältnis von 1 : 10 bis 10 : 1 vorliegen. Wasser sowie wässrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genusssäuren verwendet. Unter Genusssäuren werden solche Säuren verstanden, die im Rahmen der üblichen Nahrungsaufnahme aufgenommen werden und positive Auswirkungen auf den menschlichen Organismus haben. Genusssäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin sowie N,N,N',N'-Tetrakis-(2-hydroxypropyl)-ethylendiamin.

Die Mittel können als Einkammersystem oder als Zweikammersystem konfektioniert werden.

Neben der erfindungsgemäßen zwingend erforderlichen Wirkstoffen können die Mittel prinzipiell alle weiteren, dem Fachmann für solche kosmetischen Mittel bekannten Komponenten enthalten.

Gemäß einer besonders bevorzugten Ausführungsform enthält die erfindungsgemäßen Mitteleinen haarwuchsstimulierenden Wirkstoff. Insbesondere bevorzugt werden als haarwuchsstimulierende Wirkstoffe solche Verbindungen eingesetzt die ausgewählt sind aus 5-α-Reduktaseinhibitoren, Minoxidil (6-Piperidino-2,4-pyrimidindiamin-3-oxid) und Aminexil (Diaminopyrimidinoxid).
Als 5-α-Reduktaseinhibitoren sind insbesondere funktionellen C₂-C₁₂-Carbonsäuren und deren physiologisch verträglichen Metallsalzen, insbesondere 10-Hydroxydecansäure, 10-Hydroxydecensäure und ihren Derivaten, Derivaten von C₃-C₉-Polyolen, Phenolderivaten, Pflanzenextrakten, Riechstoffen, Flavonoiden, Isoflavonoiden, 6,7-disubstituierten 2,2-Dialkylchromanen oder -chromenen, Aluminiumchlorohydrat, 2-Phenylethanol, Etidronsäure, 7-Acetyl-1,1,3,4,4,6-hexamethyltetralin, Tropolonderivaten, Estern der Schwefelsäure mit alkoxylierten C₈-C₁₈-Fettalkoholen und deren physiologisch verträglichen Metallsalzen, Estern der Phosphorsäure und der Triphosphorsäure mit ein- bis sechswertigen Hydroxyverbindungen, Kieselsäureestern, aus marinen Organismen isolierbaren mycosporin-ähnlichen Aminosäuren (MAA) sowie quaternären Siliconverbindungen.
Unter Derivaten sind insbesondere deren Salze, Ester und Amide zu verstehen.
Ganz besonders bevorzugt sind dabei 10-Hydroxydecansäure, 10-Hydroxydecensäure und Finasterid (*N-tert*-Butyl-3-oxo-4-aza-5α-androst-1-en-17β-carboxamid) und deren Derivate.

Es wurde gefunden, dass die haarwuchsstimulierende Wirkung der Wirkstoffe durch ihren Einsatz in erfindungsgemäßen Mitteln noch verbessert werden kann. Besonders bevorzugt sind erfindungsgemäße Mittel, die zusätzlich mindestens einem Wirkstoff ausgewählt aus 10-Hydroxydecansäure, 10-Hydroxydecensäure, Minoxidil und Finasterid enthalten. Ganz besonders bevorzugt ist der haarwuchsstimulierende Wirkstoff auch in dieser Kombination ausgewählt aus Minoxidil und Finasterid.

Weitere Wirk-, Hilfs- und Zusatzstoffe sind beispielsweise
- nichtionogene Tenside wie beispielsweise Alkylphenolpolyglycolether, Fettsäurepolyglycolester, Fettsäureamidpolyglycolether, Fettaminpolyglycolether, alkoxylierte Triglyceride, wie insbesondere ethoxyliertes Rizinusöl, Alk(en)yloligoglucoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle oder eingeengte Homologenverteilung aufweisen.
- anionische Tenside, insbesondere Alkylsulfate, Alkylpolyglykolethersulfate und Ethercarbonsäuren mit 10 bis 18 C-Atomen in der Alkylgruppe und bis zu 12 Glykolethergruppen im Molekül, Seifen sowie Sulfobernsteinsäuremono- und
   -dialkylester mit 8 bis 18 C-Atomen in der Alkylgruppe und Sulfobernsteinsäuremono-alkylpolyoxyethyl-ester mit 8 bis 18 C-Atomen in der Alkylgruppe und 1 bis 6 Oxyethylgruppen,
- zwitterionische Tenside, insbesondere die sogenannten Betaine wie die N-Alkyl-N,N-dimethylammonium-glycinate, beispielsweise das Kokosalkyl-dimethylammonium-glycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat,
- ampholytische Tenside wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkyl-aminoessigsäuren mit jeweils etwa 8 bis 18 C-Atomen in der Alkylgruppe,
- nichtionische Polymere wie beispielsweise Vinylpyrrolidon/Vinylacrylat-Copoly mere, Polyvinylpyrrolidon und Vinylpyrrolidon/Vinylacetat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyln-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- Fettalkohole, insbesondere lineare und/oder gesättigte Fettalkohole mit 8 bis 30 C-Atomen, und Monoester der Fettsäuren mit Alkoholen mit 6 bis 24 C-Atomen,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie Phospholipide, beispielsweise Sojalecithin, Ei-Lecithin und Kephaline,
- quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- weitere Substanzen zur Einstellung des pH-Wertes, wie beispielsweise α- und β-Hydroxycarbonsäuren
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien.

Die erfindungsgemäßen Mittel können außerdem Tenside enthalten. Bei diesen kann es sich sowohl um anionische, ampholytische, zwitterionische oder nichtionogene Tenside als auch um kationische Tenside handeln.

In einer bevorzugten Ausführungsform der vorliegenden Erfindung wird, beispielsweise in einem Shampoo, eine Kombination aus anionischen und nichtionischen Tensiden oder eine Kombination aus anionischen und amphoteren Tensiden eingesetzt. In einem Haartonic kann der Fachmann jedoch auch weitgehend oder vollständig auf den Einsatz von Tensiden verzichten.

Es hat sich in Einzelfällen als vorteilhaft erwiesen, die Tenside aus amphoteren oder nichtionischen Tensiden auszuwählen.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.
Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der vorliegenden Erfindung ist die nicht therapeutische Verwendung, insbesondere die kosmetische Verwendung zur Vitalisierung von Haaren, Anregung des Energiestoffwechsel in Haarfollikeln, Aktivierung von Haarfollikeln, Förderung oder Verstärkung des Haarwuchses, Haarverdickung, Behandlung von Haarausfall und zur Beeinflussung (insbesondere Anregung) der Keratinsynthese, bzw. zur Haarkonditionierung.

Ganz besonders bevorzugt ist die Verwendung eines Mittels enthaltend L-Carnitintartrat, Presssaft aus Echinacea purpurea herb. und Taurin und/oder dessen Derivate.

Ein weiterer Gegenstand der vorliegenden Erfindung ist die nicht therapeutische Verwendung, insbesondere die kosmetische Verwendung, zur Verdickung epithelialer Zellen und Zellschichten, insbesondere auf der Haut, Verbesserung der Festigkeit der Haut, Stärkung der Epidermis, Verminderung des Dünner Werdens der Haut, insbesondere der Bekämpfung von Alterserscheinungen der Haut, Reduktion des transepidermalen Wasserverlustes der Haut, Verbesserung der Hautfeuchte, Schutz der Haut vor Infektionen, exogenen Faktoren wie Smog, Zigarettenrauch sowie gegen die Beanspruchung durch Tenside und/oder häufigen Wasserkontakt.

Ganz besonders bevorzugt ist die Verwendung eines Mittels enthaltend L-Carnitintartrat, einem Wirkstoff ausgewählt aus Presssäften und Extrakten, die aus Echinacea purpurea gewonnen werden, insbesondere bevorzugt Presssaft aus Echinacea purpurea, und Taurin.

Ein weiterer Gegenstand der vorliegenden Erfindung ist ein nicht therapeutisches Verfahren, insbesondere ein kosmetisches Verfahren, zur Vitalisierung von Haaren, Haarverdickung, Anregung der Keratinsynthese, Anregung des Energiestoffwechsel in Haarfollikeln, Aktivierung von Haarfollikeln, Förderung oder Verstärkung des Haarwuchses bzw. zur Haarkonditionierung, dadurch gekennzeichnet, dass man ein erfindungsgemäßes Mittel auf die Haare bzw. die behaarte Haut aufbringt.

Ein weiterer Gegenstand der vorliegenden Erfindung ist ein nicht therapeutisches insbesondere ein kosmetisches Verfahren, zur Verdickung epithelialer Zellen und Zellschichten, insbesondere auf der Haut, Verbesserung der Festigkeit der Haut, zur Stärkung der Epidermis, Verminderung des dünner Werdens der Haut, insbesondere der Haut, Reduktion des transepidermalen Wasserverlustes der Haut, Verbesserung der Hautfeuchte, Schutz der Haut vor Infektionen, exogenen Faktoren wie Smog, Zigarettenrauch sowie gegen die die Beanspruchung durch schädigende und/oder reizende Stoff, insbesondere Tenside und/oder häufigen Wasserkontakt, dadurch gekennzeichnet, dass man ein erfindungsgemäßes Mittel auf die Haut aufbringt.

Ganz besonders bevorzugt wird ein solches Verfahren mit einem Mittel enthaltend L-Carnitintartrat, Taurin und Presssaft aus Echinacea purpurea herb durchgeführt.
Die folgenden Beispiele erläutern die Erfindung, ohne sie jedoch darauf einzuschränken:
Alle Angaben sind in Gewichtsprozent (w%).

Informationen zu den in den Beispielen eingesetzten Stoffen:

### Presssaft aus Echinacea purpurea

Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt, Feststoff

### Gluadin WQ

Cognis Deutschland GmbH,
AQUA (WATER), LAURDIMONIUM HYDROXYPROPYL HYDROLYZED WHEAT PROTEIN, ETHYLPARABEN,METHYLPARABEN
Protein hydrolyzates, wheat germ, (3-(dodecyldimethylammonio)-2-hydroxypropyl), chlorides ca. 30-35 % Gehalt

### Gluadin WLM

Cognis Deutschland GmbH
Weizenproteinhydrolysat in H2O
INCI declaration [INCI] HYDROLYZED WHEAT PROTEIN
Gehalt ca. 20-24 %

### Salcare SC 96

Ciba
INCI declaration [INCI] POLYQUATERNIUM-37, PROPYLENE GLYCOL
DICAPRYLATE/DICAPRATE,
Gehalt ca. 50 %

### Cetiol HE

Cognis Deutschland GmbH
Kokosmonoglycerid ethoxyliert (7 EO)
INCI declaration [INCI] PEG-7 GLYCERYL COCOATE

### Sepigel 305

Seppic (Interorgana)
INCI declaration [INCI] POLYACRYLAMIDE, C13-14 ISOPARAFFIN, LAURETH-7 Gehalt ca. 45-50 %

### Euperlan PK 3000

Cognis Deutschland GmbH
INCI declaration [INCI] GLYCOL DISTEARATE, GLYCERIN, LAURETH-4, COCAMIDOPROPYL BETAINE

### Plantacare 818 UP

Cognis Deutschland GmbH
C8-16 Alkylpolyglucosid
*NLP
COCO-GLUCOSIDE, AQUA (WATER)

### Ajidew NL 50

Ajinomoto
Pyrrolidoncarbonsäure Natrium Salz
Na-PCA
SODIUM PCA

### Uvinul MS 40

BASF
Hydroxy-4-methoxybenzophenon-5-sulfonsäure *2-BENZOPHENONE-4

### Arlypon F

Cognis Deutschland GmbH
Lauromacrogol JP 12 (Pharmacopoe of Japan)
*NLP
C12-14 Fettalkohole ethoxyliert (2.5 EO)
LAURETH-2

### Antil 171

Goldschmidt (Degussa)
Polyol Fettsäure Ester
PEG-18 GLYCERYL OLEATE/COCOATE

### Synthalen K

Synthalen KD (alt)
3V Sigma
Polyacrylsäure
CARBOMER

### Cremophor RH 40

BASF
Riechstoff C 041
Rizinusöl, gehärtet, ethoxyliert (45 EO)
PEG-40 HYDROGENATED CASTOR OIL

### Neutrol TE

BASF
Tetrakis-(2-hydroxypropyl)-ethylendiamin *N,N,N',N',-Edetol
TETRAHYDROXYPROPYL ETHYLENEDIAMINE

### Beispiel 1:

| Haarspülung | |
|---|---|
| L-Carnitin | 2,0 |
| Taurin | 1,0 |
| Echinacea purpurea Presssaft (Flachsmann) | 0,1 |
| Eumulgin® B2¹ | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart^{®}A-CA² | 2,0 |
| Gluadin WQ | 0,2 |
| Gluadin WLM | 0,5 |
| Pantolacton | 0,5 |
| Subtilisin oder Papain | 0,5 |
| Phenonip®³ | 0,8 |
| Wasser | ad 100 |

| | |
|---|---|
| PH = 7,0 ¹ Cetylstearylalkohol + 20 EO (INCI-Bezeichnung: Ceteareth-20) (HENKEL) ² Trimethylhexadecylammoniumchlorid (ca. 25 % Aktivsubstanz in Wasser; INCI-Bezeichnung: Aqua, Cetrimonium Chloride) ³ Hydroxybenzoesäuremethylester-Hydroxybenzoesäureethylester-Hydroxybenzoesäurepropylester-Hydroxybenzoesäurebutylester-Phenoxyethanol-Gemisch (ca. 28 % Aktivsubstanz; INCI-Bezeichnung: Phenoxyethanol, Methylparaben, Ethylparaben, Propylparaben, Butylparaben) (NIPA) | |

### Beispiel 2:

| Haarspülung | |
|---|---|
| Acetyl-L-Carnitin | 1,0 |
| Echinacea purpurea Presssaft (Flachsmann) | 0,1 |
| Taurin | 0,5 |
| Eumulgin® B2 | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart®L 80⁵ | 2,0 |
| Gluadin WQ | 0,5 |
| Gluadin WLM | 0,2 |
| Pantolacton | 1,0 |
| Subtilisin oder Papain | 1,0 |
| Citronensäure | 0,4 |
| Phenonip^{®} | 0,8 |
| Wasser | ad 100 |

| | |
|---|---|
| PH - 7,0 ⁵ Bis(Cocoylethyl)-hydroxyethyl-methyl-ammonium-methosulfat (ca. 76 % Aktivsubstanz in Propylenglykol; INCI-Bezeichnung: Dicocoylethyl Hydroxyethylmonium Methosulfat, Propylene Glycol) (HENKEL) | |

### Beispiel 3:

| Haarspülung | |
|---|---|
| L-Carnitin-Tartrat | 2,0 |
| Echinacea purpurea Presssaft (Flachsmann) | 0,5 |
| Taurin | 2 |
| Isopropylmyristat | 0,50 |
| Paraffinum Liquidum | 0,50 |
| Cetearyl Alcohol | 2,5 |
| Eumulgin B 2 * | 0,40 |
| Citronensäure | 0,20 |
| Propylparaben | 0,20 |
| Wasser, vollentsalzt | ad 100 |
| Phenoxyethanol, rein | 0,30 |
| Methylparaben | 0,20 |
| Dehyquart F 75 | 2,0 |

| | |
|---|---|
| * Eumulgin B 2 = Ceteareth-20 | |

### Beispiel 4:

| Haarkur (rinse off) | |
|---|---|
| L-Carnitin-Fumarat | 4,0 |
| N-Monomethyltaurin | 1 |
| Echinacea purpurea Presssaft (Flachsmann) | 0,25 |
| Dehyquart^{®} F75⁷ | 4,0 |
| Cetyl/Stearylalkohol | 4,0 |
| Paraffmöl perliquidum 15 cSt. DAB 9 | 1,5 |
| Dehyquart^{®} A-CA | 4,0 |
| Salcare^{®}SC 96 | 0,5 |
| Gluadin WQ | 1,0 |
| Gluadin WLM | 1,0 |
| Pantolacton | 0,5 |
| Subtilisin oder Papain | 0,2 |
| Citronensäure | 0,15 |
| Phenonip® | 0,8 |
| Wasser | ad 100 |

| | |
|---|---|
| PH = 7,0 ⁷ Fettalkohole-Methyltriethanolammoniummethylsulfatdialkylester-Gemisch (INCI-Bezeichnung: Distearoylethyl Hydroxyethylmonium Methosulfate, Cetearyl Alcohol) (Cognis) | |

### Beispiel 5:

| Haarkur (rinse off) | |
|---|---|
| L-Carnitin-Citrat, | 2,0 |
| Extrakt aus Echinacea angustifolia | 0,2 |
| Taurin | 1,5 |
| Dehyquart® L80 | 4,0 |
| Cetyl/Stearylalkohol | 6,0 |
| Paraffmöl perliquidum 15 cSt. DAB 9 | 2,0 |
| Rewoquat®W 75⁹ | 2,0 |
| Sepigel®305 | 0,5 |
| Gluadin WQ | 0,2 |
| Gluadin WLM | 0,5 |
| Pantolacton | 0,5 |
| Subtilisin oder Papain | 0,5 |
| Citronensäure | 0,15 |
| Phenonip® | 0,8 |
| Wasser | ad 100 |

| | |
|---|---|
| PH = 7,0 ⁹ I-Methyl-2-nortalgalkyl-3-talgfettsäureamidoethylimidazolinium-methosulfat (ca. 75 % Aktivsubstanz in Propylenglykol; INCI-Bezeichnung: Quaternium-27, Propylene Glycol) (WITCO) | |

### Beispiel 6:

| Haarkur (auf dem Haar verbleibend) | |
|---|---|
| Lauroyl-L-Carnitin | 3,0 |
| Echinacea purpurea Presssaft (Flachsmann) | 0,2 |
| Taurin | 1,7 |
| Dehyquart^{®} F75 | 0,3 |
| Salcare^{®}SC 96 | 5,0 |
| Dow Corning^{®}200 Fluid, 5 cSt.¹⁰ | 1,5 |
| Gafquat^{®}755N¹¹ | 1,5 |
| Biodocarb^{® 12} | 0,8 |
| Gluadin WQ | 0,2 |
| Gluadin WLM | 0,5 |
| Pantolacton | 0,5 |
| Subtilisin oder Papain | 0,5 |
| Parfumöl | 0,25 |
| Wasser | ad 100 |

| | |
|---|---|
| PH = 7,0 ¹⁰ Polydimethylsiloxan (INCI-Bezeichnung: Dimethicone) (DOW CORNING) ¹¹ Dimethylaminoethylmethacrylat-Vinylpyrrolidon-Copolymer, mit Diethylsulfat quaterniert (19 % Aktivsubstanz in Wasser; INCI-Bezeichnung: Polyquaternium-11) (GAF) ¹² 3-lod-2-proinyl-n-butylcarbamat (INCI-Bezeichnung: lodopropynyl Butylcarbamate) (MILKER & GRÜNING) | |

### Beispiel 7:

| Haarkur (auf dem Haar verbleibend) | |
|---|---|
| L-Carnitin-Tartrat | 3,0 |
| Taurin | 0,9 |
| Echinacea purpurea, ethanolischer Extrakt, Feststoff | 0,2 |
| Sepigel®305 | 5,0 |
| Dow Corning®Q2-5220 5 cSt.¹³ | 1,5 |
| Genamin®DSAC¹⁴ | 0,3 |
| Phenonip® | 0,8 |
| Gluadin WQ | 0,5 |
| Gluadin WLM | 0,8 |
| Pantolacton | 1,0 |
| Subtilisin oder Papain | 0,8 |
| Parfümöl | 0,25 |
| Wasser | ad 100 |

| | |
|---|---|
| PH = 7,0 ¹³ Silicon-Glykol-Copolymer (INCI-Bezeichnung: Dimethicone Copolyol) (DOW CORNING) ¹⁴ Dimethyldistearylammoniumchlorid (INCI-Bezeichnung: Distearyldimonium Chloride) (CLARIANT) | |

### Beispiel 8:

| Haarkur | |
|---|---|
| L-Carnitin-Tartrat | 2,0 |
| N,N-Dimethyltaurin | 1,0 |
| Echinacea purpurea Presssaft (Flachsmann) | 0,2 |
| Cetearyl Alcohol | 5,00 |
| Propylparaben | 0,20 |
| Stearamidopropyldimethylamine | 1,50 |
| Dehyquart F 75 ¹⁵ | 1,50 |
| Paraffinum Liquidum | 1,00 |
| Quaternium-87 in Propylenglycol | 1,50 |
| Isopropylmyristat | 2,00 |
| Cutina GMS ¹⁶ | 1,00 |
| Methylparaben | 0,20 |
| Wasser | ad 100 |
| Citronensäure | 0,45 |
| Dehyquart A CA | 5,00 |
| Rheocare CTH (E) | 0,50 |
| Polymer JR 400 | 0,20 |
| Pantolacton | 0,20 |
| Nikotinsäureamid | 0,10 |
| Phenoxyethanol, rein | 0,40 |
| D-Panthenol 75 % | 0,20 |
| Dow Corning 1403 Fluid | 1,50 |
| Parfum | 0,20 |

| | |
|---|---|
| ¹⁵ Dehyquart F 75 = Distearoylethyl Hydroxyethylmonium Methosulfate ¹⁶ Cutina GMS = Glyceryl Monostearate | |

### Beispiel 9:

| Shampoo: | |
|---|---|
| L-Carnitin-Tartrat | 1,5 |
| Taurin | 0,5 |
| Echinacea purpurea Presssaft (Flachsmann) | 0,05 |
| LAURETHSULFAT 25% | 40 |
| CITRONENSÄURE | 0,1 |
| NATRIUMBENZOAT | 0,5 |
| DISODIUM COCOAMPHODIACETATE | 6,0 |
| SALICYLSÄURE | 0,1 |
| HYDROTRITICUM WQ | 1,0 |
| Gluadin WQ | 0,2 |
| Gluadin WLM | 0,5 |
| Pantolacton | 0,5 |
| Subtilisin oder Papain | 0,2 |
| CETIOL HE | 0,5 |
| PARFÜM | 0,4 |
| NACL | 0,5 |
| WASSER | AD 100 |

### Beispiel 10:

| Shampoo: | |
|---|---|
| L-Carnitin-Citrat | 2,0 |
| Extrakt aus Echinacea pallida | 0,2 |
| N, N-Diethyltaurin | 1,3 |
| LAURETHSULFAT 25%(ALKALISCHE VERDÜNNUNG) | 25,0 |
| CITRIC ACID MONO REGULÄR | 0,3 |
| TIMIRON | 0,5 |
| NATRIUMBENZOAT | 0,5 |
| PANTHENOL 75 L | 0,2 |
| EUPERLAN PK 3000 | 8,0 |
| PLANTACARE 818 UP | 2,0 |
| UVINUL MS40 | 1,0 |
| SALICYLSAEURE | 0,2 |
| AJIDEW NL-50 | 2,0 |
| CUTINA HR GEMAHLEN | 0,5 |
| CETIOL HE | 1,0 |
| CITRIC ACID MONO REGULÄR | 0,03 |
| JAGUAR EXCEL | 0,3 |
| Gluadin WQ | 0,2 |
| Gluadin WLM | 0,5 |
| Pantolacton | 0,5 |
| Subtilisin oder Papain | 0,5 |
| NATRIUMCHLORID | 0,3 |
| WASSER | ad 100 |

### Beispiel 11:

| Shampoo: | |
|---|---|
| L-Carnitin | 2,0 |
| Echinacea purpurea Presssaft (Flachsmann) | 0,5 |
| TAURIN | 1 |
| LAURETHSULFAT 25%(ALKALISCHE VERDÜNNUNG) | 50 |
| CITRIC ACID MONO REGULÄR | 0,4 |
| ARLYPON F | 0,5 |
| ANTIL171 | 0,3 |
| WEIZENPROTEINHYDROLYSAT KATIONISIERT | 1,5 |
| NATRIUMBENZOAT | 0,5 |
| EUPERLAN PK 3000 | 6,0 |
| COCAMIDOPROPYL BETAINE 45 % | 5,0 |
| SALICYLSAEURE | 0,2 |
| SILSOFT A-858 | 0,3 |
| Gluadin WQ | 1,0 |
| Gluadin WLM | 1,0 |
| Pantolacton | 0,5 |
| Subtilisin oder Papain | 0,2 |
| CUTINA HR | 0,2 |
| CETIOL HE | 1,0 |
| WASSER | ad 100 |

### Beispiel 12:

| Shampoo: | |
|---|---|
| L-Carnitin-Tartrat | 1,0 |
| Echinacea purpurea Presssaft (Flachsmann) | 0,05 |
| Taurin | 1,0 |
| Vorl. Laurethsulf.25% alk.Ver. | 40,00 |
| Citronensäure | 0,15 |
| Disodium Cocoamphodiacetate | 7,00 |
| Na-benzoat | 0,50 |
| Salicylsäure | 0,20 |
| Parfum | 0,15 |
| Natriumchlorid | 1,50 |
| Wasser, vollentsalzt | ad 100 |
| Polymer JR 400 | 0,30 |

### Beispiel 13:

| Haarstylinggel: | |
|---|---|
| Acetyl-L-Carnitin | 2,5 |
| Echinacea purpurea Presssaft (Flachsmann) | 0,5 |
| Taurin | 1 |
| SYNTHALEN K | 0,6 |
| NEUTROLTE | 0,5 |
| GLYZERIN DAB 9, 86,5 | 8,00 |
| ETHYLALKOHOL VERGÄLLT 96 VOL % FLS | 30,00 |
| Gluadin WQ | 0,5 |
| Gluadin WLM | 0,5 |
| Pantolacton | 1,0 |
| Subtilisin oder Papain | 0,2 |
| POLYETHYLENGLYKOL | 2,00 |
| PVP/VA W-635 | 6,50 |
| CREMOPHOR RH 40 | 1,00 |
| PARFÜM | 0,50 |
| ENTSALZTES WASSER | ad 100 |

| | |
|---|---|
| PH = 7,0 | |

### Beispiel 14:

| Haarspray: | |
|---|---|
| L-Carnitin-Tartrat | 2,0 |
| Taurin | 0,8 |
| Echinacea purpurea Presssaft (Flachsmann) | 0,1 |
| AMPHOMER | 3,00 |
| LUVISKOL VA 37 | 16,00 |
| AMP AMINO-METHYL-PROPANOL 100 | 0,60 |
| ISOPROPYLMYRISTAT | 0,12 |
| Gluadin WQ | 0,5 |
| Gluadin WLM | 0,5 |
| Pantolacton | 0,2 |
| Subtilisin oder Papain | 1,0 |
| PARFÜM | 0,20 |
| ENTSALZTES WASSER | ad 100 |
| ETHYLALKOHOL VERGÄLLT 96 VOL % FLS | 67,50 |

| | |
|---|---|
| PH = 7,0 | |

### Beispiel 15:

| Haartonic: | |
|---|---|
| Lauroyl-L-Carnitin | 1,0 |
| Echinacea purpurea Presssaft (Flachsmann) | 0,05 |
| Taurin | 1 |
| ENTSALZTES WASSER | ad 100 |
| PANTHENOL 75 | 0,1 |
| Gluadin WQ | 0,2 |
| Gluadin WLM | 0,5 |
| Pantolacton | 0,5 |
| Subtilisin oder Papain | 0,5 |
| CARBOPOL | 0,1 |
| NEUTROLTE | 0,10 |
| ETHYLALKOHOL VERGÄLLT 96 VOL % | 30,0 |

| | |
|---|---|
| PH = 7,0 | |

### Beispiel 16:

| Haartonic: | |
|---|---|
| L-Carnitin-Tartrat | 2,0 |
| Extrakt aus Echinacea purpurea | 0,1 |
| Taurin | 0,01 |
| D-Panthenol 75 % | 0,20 |
| Allantoin | 0,10 |
| Parfum | 0,25 |
| Wasser, vollentsalzt | ad 100 |
| Cremophor A25* | 0,2 |
| Ethanol 96 % | 35,00 |

| | |
|---|---|
| * Cremophor A25* = Ceteareth-25 | |

### Beispiel 17:

### Haarspülung wie in Bsp. 1, als 2-K System :

| 1. Kammer: | |
|---|---|
| Echinacea purpurea Presssaft (Flachsmann) | 0,6 |
| Eumulgin® B2¹ | 0,3 |
| Cetyl/Stearylalkohol | 3,3 |
| Isopropylmyristat | 0,5 |
| Paraffmöl perliquidum 15 cSt. DAB 9 | 0,3 |
| Dehyquart®A-CA² | 2,0 |
| Gluadin WQ | 0,2 |
| Gluadin WLM | 0,5 |
| Pantolacton | 0,5 |
| Phenonip®³ | 0,8 |
| Wasser | ad 100 |

| | |
|---|---|
| PH = 4,0 | |

| 2. Kammer | |
|---|---|
| L-Carnitin | 2,0 |
| Taurin | 1,0 |
| Wasser | ad 100 |

### Beispiel 18:

### Haarkur analog Bsp. 7, aber in zwei Anwendungsschritten:

| Vorbehandlung mit L-Carnitin-Tartrat und Enzym | |
|---|---|
| L-Carnitin-Tartrat | 3,0 |
| Subtilisin oder Papain | 0,5 |
| oder Papayaextrakt | 1,0 |
| Wasser | ad 100 |

| Nachbehandlung | |
|---|---|
| Sepigel®305 | 5,0 |
| Dow Corning®Q2-5220 5 cSt.¹³ | 1,5 |
| Genamin®DSAC¹⁴ | 0,3 |
| Phenonip® | 0,8 |
| Gluadin WQ | 0,5 |
| Gluadin WLM | 0,8 |
| Pantolacton | 1,0 |
| Parfümöl | 0,25 |
| Taurin | 1 |
| Echinacea purpurea Presssaft (Flachsmann) | 0,5 |
| Wasser | ad 100 |

### Beispiel 19:

| Haarkur (Leave-on) | |
|---|---|
| Glycerylmonooleat | 0,50 |
| Methylparaben | 0,30 |
| Milchsäure 80%ig | 0,20 |
| Dehyquart A CA | 4,00 |
| Dehyquart L 80 | 5,00 |
| Pantolacton | 0,40 |
| Phenoxyethanol, rein | 0,50 |
| D-Panthenol 75 % | 0,20 |
| Nutrilan Keratin W¹⁷ | 0,50 |
| Dow Corning 1403 Fluid¹⁸ | 1,50 |
| Gluaden WLM | 0, 50 |
| Parfum | 0,20 |
| Wasser | ad 100 |
| L-Carnitin-Tartrat | 2,0 |
| Taurin | 1,0 |
| Echinacea purpurea Presssaft (Flachsmann) | 0,05 |

| | |
|---|---|
| ¹⁷ Nutrilan Keratin W (22%ige Aktivsubstanz in Wasser) = Keratinhydrolysat (Cognis) ¹⁸ Dow Corning 1403 Fluid = Dimethicone, Dimethiconol (Dow Corning) | |

### Beispiel 20: Bestimmung der ATP-Syntheserate

ATP (Adenosintriphosphat) ist die universelle Speicherform für chemische Energie in Zellen. Bei der Abspaltung der distalen Phosphatgruppe entsteht ADP und Pᵢ (anorganisches Phosphat). Diese Reaktion ist stark exergon, d.h. es wird Energie frei. Produziert wird ATP beim zellulären, oxidativen Abbau von Fetten, Kohlehydraten und Proteinen. Es dient als Energielieferant für biochemische Synthesen, für Transportvorgänge (aktiver Transport) und für mechanische Arbeit. Diese Vorgänge sind endergon, d.h. sie laufen nur unter Energiezufuhr ab. Um ihren Stoffwechsel optimal aufrecht zu erhalten, sind Zellen also auf eine ausreichende Versorgung mit ATP angewiesen. Auch dermale Papillenzellen benötigen beispielsweise zur Produktion von Wachstumsfaktoren und damit zur Steuerung des Haarzyklus ATP. Die Proliferation und Differenzierung von ORS Keratinozyten ist ebenfalls an die ATP-Synthese gekoppelt, da für beide Vorgänge die Biosynthese spezifischer Proteine essentielle Voraussetzung ist. Kann durch ein Produkt die ATP-Syntheserate der haarrelevanten Zellen erhöht werden, so steht den Zellen mehr Energie zur Verfügung um Stoffwechselvorgänge und zelluläre Strukturen aufrecht zu erhalten, und um Strukturen zu erneuern, z.B. bei Reparaturprozessen oder dem Neuaufbau von Haaren.

### ATP-Nachweismethode

Die ATP-Bestimmungen erfolgten mit Hilfe des ATPLiteTM-M Assays (Packard). Das Testprinzip dieses Assays beruht darauf, dass die Luciferase von Photinus pyralis eine Reaktion katalysiert, bei der in Gegenwart von ATP D-Luciferin in Oxyluciferin umgewandelt wird. Bei dieser Reaktion wird grünes Licht emittiert, das mit einem Luminometer gemessen werden kann. Das emittierte Biolumineszenzlicht ist proportional zur Menge des vorhandenen ATP.

Zur Bestimmung der ATP-Aktivität in dermalen Papillenzellen werden diese in geeigneter Weise unter Erhalt ihrer spezifischen Eigenschaften vorkultiviert (DE10162814) und in eine 48well-Zellkulturschale überführt. Die Behandlung mit dem Substanzgemisch erfolgte über 24 Stunden gegen eine unbehandelte Kontrolle. Anschließend wurden die Zellen mit jeweils 100 µl/Kavität eines im Testkit enthaltenen Lysepuffer für 5 min auf einem Schüttler lysiert. Danach wurden die Zellen für weitere 5 min mit jeweils 100 µl/Kavität mit der mitgelieferten Substratlösung auf dem Schüttler inkubiert und anschließend das Reaktionsgemisch in eine schwarze Mikrotiterplatte überführt. Nach einer Inkubationszeit von 10 min in der Dunkelheit erfolgte die Messung der Lumineszenz.

Die Substanzmischung aus L-Carnitintartrat (0,01%), Echinacea purpurea [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] (0,1%) und Taurin (0,01%) steigerte die ATP-Produktion der dermalen Papillenzellen gegenüber der unbehandelten Kontrolle um 65% (Tabelle 1).

**Tabelle 1: Einfluss der Substanzmischungen auf die ATP-Produktion von demalen Papillenzellen in % (Standardabweichung)**

| | Mittelwert |
|---|---|
| Unbehandelt | 100 |
| Substanzmischung | 165 (28) |

### Beispiel 21: Nachweis der Produktion von haarrelevanten Wachstumsfaktoren

Hepatocyte Growth Factor (HGF) und Keratinocyte Growth Factor (KGF) sind wichtige Wachstumsfaktoren, die von der dermalen Papille ausgeschüttet werden, um die Proliferation der Haar-Keratinozyten zu steuern. Eine potentiell wachstumsfördernden und das Haar stärkenden Substanz kann von einer Steigerung der ins Medium ausgeschütteten Faktoren ausgegangen werden. Die Ausschüttung von HGF sowie KGF kann mit Hilfe von kommerziell erhältlichen ELISA-Kits quantifiziert werden. Dazu werden organotypische Zellkulturen über 72h mit der Substanzmischung inkubiert und die Konzentration der Wachstumsfaktoren im Medium in beschriebener Weise bestimmt.

Die Substanzmischung aus L-Carnitintartrat (0,01%), Echinacea purpurea [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] (0,1%) und Taurin (0,01%) steigerte die Produktion von HGF gegenüber der unbehandelten Kontrolle um maximal 412%, die Produktion von KGF gegenüber der unbehandelten Kontrolle um maximal 40%. Ebenfalls in der Tabelle aufgeführt ist die Steigerung der Wachstumsfaktoren durch Applikation der Einzelsubstanzen, deren Summe geringer ausfällt als durch die Inkubation mit der Mischung (Tabelle 2).

**Tabelle 2: Einfluss der Substanzmischung auf die Produktion von Wachstumsfaktoren [%] (Standardabweichung)**

| | HGF Mittelwert [%] | KGF Mittelwert [%] |
|---|---|---|
| Unbehandelt | 100 | 100 |
| Substanzmischung | 512(6) | 140(10) |
| Echinacea 0,1% | 445 (28) | 96 (9) |
| Taurin 0,01% | 120 (21) | 71 (7) |
| Carnitintartrat 0,01% | 100 (42) | 108 (8) |

### Beispiel 22: Steigerung der Keratinsynthese

Die Haarstruktur ist im Wesentlichen von der Zusammensetzung spezieller haarspezifischer Strukturproteine abhängig, den Haarkeratinen. Durch die Beeinflussung der Zusammensetzung dieser spezifischen Proteine kann auf biologischer Ebene Einfluss auf die Haarstruktur genommen werden.
Die Expression verschiedener Haarkeratine im organotypischen Modell kann mit Hilfe eines quantitativen Real-Time-PCR-Verfahrens untersucht werden. Zur Durchführung der PCR wird zunächst mit Hilfe des RNeasy Mini Kits der Fa. Qiagen die RNA aus den organotypischen Modellen isoliert und mittels reverser Transkription in cDNA umgeschrieben. Bei der anschließenden PCR Reaktion, die mit Hilfe genspezifischer Primer für die jeweiligen Haarkeratine durchgeführt wird und die der Amplifikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens ist, desto größer ist die Menge an gebildetem PCR-Produkt und um so höher ist das Fluoreszenzsignal.

Zur Quantifizierung der Genexpression wird die unbehandelte Kontrolle gleich 1 gesetzt und die Expression der zu bestimmenden Gene darauf bezogen (x-fache Expression). Dabei werden Werte, die größer/gleich der 1,8fachen Expression der unbehandelten Kontrolle sind als signifikant eingestuft.

Die Substanzmischung aus L-Carnitintartrat (0,01%), Echinacea purpurea [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] (0,1%) und Taurin (0,01%) steigerte die Genexpression der Haarkeratine hHa3-I und hHa4 im organotypischen Modell gegenüber der unbehandelten Kontrolle um den Faktor 3,5 (Tabelle 3).

**Tabelle 3: Einfluss der Substanzmischung auf die Keratinsynthese**

| | hHa3-I | hHa4 |
|---|---|---|
| Unbehandelt | 1 | 1 |
| Substanzmischung | 3,5 | 3,5 |

### Beispiel 23: Nachweis verschiedener haarrelevanter Marker mittels DNA Array

Um die Wirkung des Wirkstoffgemisches umfassend zu charakterisieren, wurden organotypische Zellkulturen für 6h und 24h mit der Wirkstoffmischung aus L-Carnitintartrat (0,01%), Echinacea purpurea [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] (0,1%) und Taurin (0,01%) behandelt, die RNA isoliert und die Expression von 850 verschiedenen Markern mit Hilfe eines DNA Chip Arrays untersucht. Als Kontrolle wurden unbehandelte Zellkulturen mitgeführt. Sowohl nach 6h als auch nach 24h wurde für verschiedene haarrelevante Parameter eine differentielle Genregulation nachgewiesen. Dabei wurden nach 6h insbesondere Wachstumsfaktoren und Proliferations- also Zellteilungsmarker hochreguliert, nach 24h solche Gene, die auf eine Aktivierung des Stoffwechsels sowie eine Induktion der Keratinsynthese und der Produktion extrazellulärer Matrix hinweisen (Tabelle 4).

Zur Quantifizierung der Genexpression wird die unbehandelte Kontrolle gleich 1 gesetzt und die Expression der zu bestimmenden Gene darauf bezogen (x-fache Expression). Dabei werden Werte, die größer/gleich der 1,7fachen Expression der unbehandelten Kontrolle sind als statistisch auffällig, Werte, die größer/gleich der 1,9fachen Expression der unbehandelten Kontrolle sind als signifikant eingestuft.

**Tabelle 4:**

| Differenzielle Genexpression nach Behandlung mit der Substanzmischung (unbehandelte Kontrolle =1) | | |
|---|---|---|
| | Expression 6h | Expression 24h |
| Wachstum/Proliferation | | |
| Ki 67 | 1,85 | -1,29 |
| IGF2 | 1,63 | -1,29 |
| cmyc | 2,02 | -1,28 |
| IER2 | 1,91 | -1,21 |
| EGF | -1,15 | 2,04 |

| Stoffwechsel | | |
|---|---|---|
| Glucosetransporter Typ I | -1,07 | 2,00 |
| Hexokinase Typ II | -1,18 | 2,50 |
| Lactatdehydrogenase | -2,13 | 2,17 |
| Glutamatdehydrogenase II | -1,56 | 2,17 |
| Ornithindecarboxylase | 1,63 | 1,75 |
| Glutaminsythetase | 1,46 | 1,96 |
| Putative Adenosylhomocysteinase | -1,20 | 2,27 |

| Extrazelluläre Matrix | | |
|---|---|---|
| Laminin alpha2 | 1,42 | 1,85 |
| Laminin alpha 3 | 1,14 | 3,23 |
| Laminin gamma 2 | 1,08 | 1,92 |
| Collagen 11 | 1,07 | 2,44 |
| Collagen 17 | -1,6 | 3,45 |

Die Expressionssteigerung im Bereich der Wachstumsfaktoren und Proliferationsmarker deuten auf ein das Haarwachstum förderndes Potential des Substanzgemisches hin, die Steigerung der Expression extrazellulärer Matrix-Gene auf einen positiven Einfluss auf die Verankerung des Haares in der Kopfhaut, da der Haarfollikel in der Kopfhaut von collagen- und lamininhaltigen Zellschichten umgeben ist. Die Anregung der Wachstumsfaktoren beeinflusst darüber hinaus auch die Haardicke positiv, da die Haardicke unter anderem von der Dicke der ORS-Zellschicht abhängt, die für die Bildung des Haarschaftes verantwortlich ist. Auch die Steigerung der Stoffwechselaktivität begünstigt das Haarwachstum, da für die zu Grunde liegenden Prozesse ausreichend Bausteine wie z.B. Aminosäuren zum Proteinaufbau bereitgestellt werden müssen; die dafür benötigte Energie wird z.B. durch die Verstoffwechselung von Glucose bereitgestellt.

### Beispiel 24: Steigerung der Epitheldicke in vitro

Da die Wachstumsfaktoren HGF und KGF die Proliferation von Epithelzellen beeinflussen, kann der stärkende Effekt der eingesetzten Prüfsubstanz auch an der Schichtdicke der dem Modell aufgelagerten ORS-Keratinozyten nachgewiesen werden. Dazu werden von jeweils drei organotypischen Modellen je drei Schnitte angefertigt, die an je fünf Stellen vermessen werden. Zur besseren Übersicht werden die histologischen Schnitte zuvor mit Propidiumiodid eingefärbt. Mit Hilfe einer Digitalkamera und einer Bildverarbeitungssoftware kann dann die Schichtdicke der einzelnen Modelle vermessen werden.

Die Substanzmischung aus L-Carnitintartrat (0,01%), Echinacea purpurea [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] (0,1%) und Taurin (0,01%) steigerte die Epitheldicke im organotypischen Modell gegenüber der unbehandelten Kontrolle um maximal 80% (Tabelle 3).

**Tabelle 5: Einfluss der Substanzmischung auf die Epitheldicke [%] (Standardabweichung)**

| | Epitheldicke [%] |
|---|---|
| Unbehandelt | 100 |
| Substanzmischung | 180 (28) |

### Beispiel 25: Verdickung des Haarfollikels in vivo

Zur Untersuchung des Einflusses der Substanzmischung auf die Dicke des Haarfollikels in vivo wurden eine in vivo Studie mit 4 Probanden durchgeführt. In einem Halbseitentest wurde ein Haartonic mit L-Carnitintartrat (2%), Echinacea purpurea [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] (0,05%) und Taurin (1%) verkapselt in Liposomen mit einer Placeboformulierung ohne Wirkstoffe (mit Liposomen) verglichen. Vor Beginn der Studie wurde bei jedem Probanden auf jeder Seite der Ausgangswert bestimmt. Dazu wurden jeweils 6 Haare gezupft und an drei verschiedenen Stellen mehrfach standardisiert vermessen. Diese Messung wurde nach einer und nach zwei Wochen in gleicher Weise wiederholt.

Nach zwei Wochen wurde bei einem nach Behandlung mit der Verumformulierung eine signifikante Verdickung des Follikels gegenüber der mit Placebo behandelten Seite nachgewiesen. Gegenüber dem Ausgangswert wurde der Haarfollikel im Bereich der äußeren Wurzelscheide nach Anwendung der Substanzmischung um rund 20% verdickt (Tabelle 6).

**Tabelle 6: Einfluss der Substanzmischung auf die Verdickung des Haarfollikels im Bereich der äußeren Wurzelscheide [%] (Standardabweichung)**

| | Ausgangswert | 1 Woche | 2 Wochen |
|---|---|---|---|
| Placebo | 100 (11) | 71 (8) | 80 (24) |
| Substanzmischung(Verum) | 100 (18) | 108 (21) | 120 (14) |

### Beispielformulierung des Haartonic:

### Rezeptur Verum:

30 % Ethanol (kosmetisch)
2% Liposomen PC (Lipoid SL80, Supplier Lipoid)
2% L-Carnitin-L-tartrat (Supplier: Loncha)
0,05% Echinacea purpurea Presssaft (Flachsmann)
1 % Taurin
ad 100 % Wasser

### Rezeptur Placebo:

30 % Ethanol (kosmetisch)
2% Liposomen PC (Lipoid SL80, Supplier Lipoid)
68% Wasser

### Beispiel 26: Bestimmung von Markern der Verdickung des Haarfollikels in vivo

Zur Untersuchung des Einflusses eines erfindungsgemäßen Wirkstoffgemisches auf die Dicke des Haarfollikels wurde eine in vivo Studie mit 14 Probanden durchgeführt. In einem Halbseitentest wurde eine Leave-on Kur gemäß Beispiel 19 mit L-Carnitintartrat (2%), Echinacea purpurea (0,05%) [Presssaft aus Echinacea purpurea (L.) Moench, Echinaceae purp. hba succ. sicc, EFLA894, Flachsmann (Artikelnr. 008594), gemäß dem von der Firma Flachsmann patentierten Verfahren (EP-B-0 730 830) hergestellt] und Taurin (1%) mit einem unbehandelten Areal am Oberkopf verglichen.

Als Parameter wurden die Expression den Proliferationsmarkers PCNA sowie die Produktion von Hepatocyte Growth Factor (HGF) und Keratinocyte Growth Factor (KGF) auf Proteinebene untersucht. HGF und KGF sind Wachstumsfaktoren, mit denen die dermale Papille das Wachstum und die Proliferation von Matrixkeratinozyten steuert. Matrixkeratinozyten sind im Bulbus lokalisiert. Aus der Dicke des Bulbus, der durch die Anzahl der vorhandenen Keratinozyten bestimmt wird, lassen sich Rückschlüsse auf die Follikeldicke und damit auf die Dicke des keratinisierten Haares ziehen.

Vor Beginn der Studie wurden den Probanden an allen Arealen jeweils 15 Haare gezupft und die Expression von PCNA sowie die Produktion von HGF und KGF bestimmt. Die Produkte wurden über den Zeitraum von einer Woche 2x täglich appliziert und täglich wurden an den entsprechenden Arealen weitere Haarproben zur Analyse entnommen.

Die Analyse der PCNA-Expression ergab einen deutlichen Vorteil der Formulierung mit L-Carnitintartrat, Echinacea purpurea und Taurin gegenüber dem unbehandelten Areal. Dieser Effekt war an Tag drei und vier nachweisbar.

**Tabelle 7: relative Expressionsänderung durch das Wirkstoffgemisch bezogen auf die unbehandelte Kontrolle (=1)**

| | Tag 1 | Tag 2 | Tag 3 | Tag 4 |
|---|---|---|---|---|
| unbehandelt | 1 | 1 | 1 | 1 |
| Testformulierung | -1,13 | 1,33 | 2,59 | 1,42 |

Zur besseren Vergleichbarkeit der Proteinausschüttung wurde in jeder Probe zusätzlich der Gesamtproteingehalt bestimmt und die Ausschüttung an HGF/KGF darauf bezogen (z.B. Menge KGF/µg Gesamtprotein). Verglichen mit dem Ausgangswert an Tag 0 ergab die Quantifizierung der KGF-Produktion einen Vorteil der Formulierung mit L-Carnitintartrat, Echinacea purpurea und Taurin gegenüber dem unbehandelten Areal am Ende der Behandlungszeit (Tag 4).

**Tabelle 8: Produktion von KGF [pg] bezogen auf das Gesamtprotein, angegeben ist der Mittelwert aller Probanden mit Standardfehler des Mittelwertes (SEM)**

| | Tag 0 | Tag 4 |
|---|---|---|
| unbehandelt | 2,5 (0,2) | 3,3 (0,6) |
| Testformulierung | 3,3 (0,3) | 5,4(1,7) |

Die Quantifizierung von HGF führte zu keiner signifikanten Differenz in dem behandelten Areal.

## Patentansprüche

1. Mittel, insbesondere zur Behandlung des Haares oder der Haut, enthaltend eine Kombination aus mindestens einer Verbindung ausgewählt aus
a) L-Carnitin, und/oder den Carnitinderivaten Acetyl-L-Carnitin, L-Carnitin-Fumarat, L-Carnitin-Citrat, Lauroyl- L-Carnitin und insbesondere L-Carnitin-Tartrat sowie
b) mindestens einer weiteren Substanz, welche ausgewählt ist aus Wirkstoffen ausgewählt aus Presssäften und Extrakten, die aus Echinacea purpurea gewonnen werden und
c) Taurin.

2. Mittel nach einem der vorstehenden Ansprüche, **dadurch gekennzeichnet, dass** es 0,001 bis 10 Gew.-% bezogen auf das gesamte Mittel, insbesondere 0,1 bis 5 Gew.-% L-Carnitin und/oder eines Carnitinderivates ausgewählt aus Acetyl-L-Carnitin, L-Carnitin-Fumarat, L-Carnitin-Citrat, Lauroyl- L-Carnitin und insbesondere L-Carnitin-Tartrat enthält.

3. Mittel nach einem der vorstehenden Ansprüche, **dadurch gekennzeichnet, dass** es 0,001 bis 10 Gew.-%, insbesondere 0,1 bis 5 Gew.-% bezogen auf das gesamte Mittel Taurin enthält.

4. Mittel nach einem der vorstehenden Ansprüche, **dadurch gekennzeichnet, dass** es 0,001 bis 10 Gew.-%, insbesondere 0,01 bis 2 Gew.-% Presssaft aus Echinacea purpurea, enthält.

5. Mittel nach einem der vorstehenden Ansprüche, **dadurch gekennzeichnet, dass** es zusätzlich einen Wirkstoff, erhältlich aus Pflanzen der Familie Apiacea, bevorzugt aus Foeniculum vulgare, enthält.

6. Mittel nach einem der vorstehenden Ansprüche, **dadurch gekennzeichnet, dass** es zusätzlich einen haarwuchsstimulierenden Wirkstoff, insbesondere ausgewählt aus Finasterid und Minoxidil enthält.

7. Nicht therapeutische Verwendung eines Mittels nach einem der Ansprüche 1 bis 6 zur Vitalisierung von Haaren, Anregung des Energiestoffwechsel in Haarfollikeln, Aktivierung von Haarfollikeln, Förderung oder Verstärkung des Haarwuchses, Haarverdickung, Behandlung von Haarausfall und zur Beeinflussung (insbesondere Anregung) der Keratinsynthese, bzw. zur Haarkonditionierung.

8. Nicht therapeutische Verwendung eines Mittels nach einem der Ansprüche 1 bis 6 zur Verdickung epithelialer Zellen und Zellschichten, insbesondere auf der Haut, Verbesserung der Festigkeit der Haut, zur Stärkung der Epidermis, Verminderung des dünner Werdens der Haut, insbesondere der Bekämpfung von Alterserscheinungen der Haut, Reduktion des transepidermalen Wasserverlustes der Haut, Verbesserung der Hautfeuchte, Schutz der Haut vor Infektionen, exogenen Faktoren wie Smog, Zigarettenrauch sowie gegen die Beanspruchung durch schädigende und/oder reizende Stoff, insbesondere Tenside und/oder häufigen Wasserkontakt.

9. Nicht therapeutisches Verfahren zur Vitalisierung von Haaren, Anregung des Energiestoffwechsel in Haarfollikeln, Aktivierung von Haarfollikeln, Förderung oder Verstärkung des Haarwuchses, Haarverdickung, Behandlung von Haarausfall und zur Beeinflussung (insbesondere Anregung) der Keratinsynthese, bzw. zur Haarkonditionierung, **dadurch gekennzeichnet, dass** man ein Mittel nach einem der Ansprüche 1 bis 6 auf die Haare bzw. die behaarte Haut aufbringt.

10. Nicht therapeutisches Verfahren zur Verdickung epithelialer Zellen und Zellschichten, insbesondere auf der Haut, Verbesserung der Festigkeit der Haut, zur Stärkung der Epidermis, Verminderung des dünner Werdens der Haut, insbesondere der Bekämpfung von Alterserscheinungen der Haut, Reduktion des transepidermalen Wasserverlustes der Haut, Verbesserung der Hautfeuchte, Schutz der Haut vor Infektionen, exogenen Faktoren wie Smog, Zigarettenrauch sowie gegen die Beanspruchung durch schädigende und/oder reizende Stoff, insbesondere Tenside und/oder häufigen Wasserkontakt, **dadurch gekennzeichnet, dass** man ein Mittel nach einem der Ansprüche 1 bis 6 auf die Haut aufbringt.

## Claims

1. An agent, in particular for treating the hair or the skin, containing a combination of at least one compound selected from
a) L-carnitine, and/or the carnitine derivatives acetyl-L-carnitine, L-carnitine fumarate, L-carnitine citrate, lauroyl-L-carnitine and in particular L-carnitine tartrate, and
b) at least one further substance that is selected from active ingredients selected from pressed juices and extracts that are obtained from *Echinacea purpurea*, and
c) taurine.

2. The agent according to one of the preceding claims, **characterized in that** it contains from 0.001 to 10 wt.% based on the total agent, in particular from 0.1 to 5 wt.% L-carnitine and/or a carnitine derivative selected from acetyl-L-carnitine, L-carnitine fumarate, L-carnitine citrate, lauroyl-L-carnitine and in particular L-carnitine tartrate.

3. The agent according to one of the preceding claims, **characterized in that** it contains from 0.001 to 10 wt.%, in particular from 0.1 to 5 wt.% taurine, based on the total agent.

4. The agent according to one of the preceding claims, **characterized in that** it contains from 0.001 to 10 wt.%, in particular from 0.01 to 2 wt.% pressed juice from *Echinacea purpurea.*

5. The agent according to one of the preceding claims, **characterized in that** it additionally contains an active ingredient that can be obtained from plants of the Apiacea family, preferably from *Foeniculum vulgare.*

6. The agent according to one of the preceding claims, **characterized in that** it additionally contains a hair-growth-stimulating active ingredient, in particular selected from finasteride and minoxidil.

7. The non-therapeutic use of an agent according to one of claims 1 to 6 for vitalizing hair, stimulating energy metabolism in hair follicles, activating hair follicles, promoting or increasing hair growth, thickening hair, treating hair loss and influencing (in particular stimulating) keratin synthesis, or for conditioning hair.

8. The non-therapeutic use of an agent according to one of claims 1 to 6 for thickening epithelial cells and cell layers, in particular on the skin, improving the firmness of the skin, strengthening the epidermis, lessening thinning of the skin, in particular countering signs of ageing of the skin, reducing transepidermal water loss of the skin, improving skin moisture, protecting the skin against infections, exogenous factors such as smog and cigarette smoke, and stress from harmful and/or irritant substances, in particular surfactants and/or frequent water contact.

9. A non-therapeutic method for vitalizing hair, stimulating energy metabolism in hair follicles, activating hair follicles, promoting or increasing hair growth, thickening hair, treating hair loss and influencing (in particular stimulating) keratin synthesis, or for conditioning hair, **characterized in that** an agent according to one of claims 1 to 6 is applied to the hair or the skin having hair.

10. A non-therapeutic method for thickening epithelial cells and cell layers, in particular on the skin, improving the firmness of the skin, strengthening the epidermis, lessening thinning of the skin, in particular countering signs of ageing of the skin, reducing transepidermal water loss of the skin, improving skin moisture, protecting the skin against infections, exogenous factors such as smog and cigarette smoke, and stress from harmful and/or irritant substances, in particular surfactants and/or frequent water contact, **characterized in that** an agent according to one of claims 1 to 6 is applied to the skin.

## Revendications

1. Agent, en particulier de traitement du cheveu ou de la peau, contenant une combinaison d'au moins un composé choisi parmi
a) la L-carnitine et/ou les dérivés de carnitine acétyl-L-carnitine, fumarate de L-carnitine, citrate de L-carnitine, lauroyl-L-carnitine et en particulier tartrate de L-carnitine ainsi que
b) au moins une autre substance qui est choisie parmi des principes actifs choisis parmi des jus pressés et des extraits, obtenus à partir d'*Echinacea purpurea*, et
c) la taurine.

2. Agent selon l'une quelconque des revendications précédentes **caractérisé en ce qu'**il contient 0,001 à 10 % en poids par rapport au total de l'agent, en particulier 0,1 à 5 % en poids de L-carnitine et/ou d'un dérivé de carnitine choisi parmi l'acétyl-L-carnitine, le fumarate de L-carnitine, le citrate de L-carnitine, la lauroyl-L-carnitine et en particulier le tartrate de L-carnitine.

3. Agent selon l'une quelconque des revendications précédentes **caractérisé en ce qu'**il contient 0,001 à 10 % en poids, en particulier 0,1 à 5 % en poids de taurine, par rapport au total de l'agent.

4. Agent selon l'une quelconque des revendications précédentes **caractérisé en ce qu'**il contient 0,001 à 10 % en poids en particulier 0,01 à 2 % en poids de jus pressé d'*Echinacea purpurea.*

5. Agent selon l'une quelconque des revendications précédentes **caractérisé en ce qu'**il contient en plus un principe actif pouvant être obtenu à partir de plantes de la famille des *Apiaceae*, de préférence à partir du *Foeniculum vulgare.*

6. Agent selon l'une quelconque des revendications précédentes **caractérisé en ce qu'**il contient en plus un principe actif stimulant la croissance des cheveux, choisi notamment entre le finastéride et le minoxidil.

7. Utilisation non thérapeutique d'un agent selon l'une quelconque des revendications 1 à 6 pour la vitalisation des cheveux, l'excitation du métabolisme énergétique dans les follicules pileux, l'activation de follicules pileux, la stimulation ou le renforcement de la croissance des cheveux, l'épaississement des cheveux, le traitement de la chute des cheveux et l'influence (notamment l'excitation) de la synthèse de la kératine ou la mise en forme des cheveux.

8. Utilisation non thérapeutique d'un agent selon l'une quelconque des revendications 1 à 6 pour l'épaississement de cellules et couches de cellules épithéliales, en particulier sur la peau, l'amélioration de la résistance de la peau, le renforcement de l'épiderme, la réduction de l'amincissement de la peau, en particulier la lutte contre les signes du vieillissement de la peau, la réduction de la perte d'eau transépidermique de la peau, l'amélioration de l'hydratation cutanée, la protection de la peau contre les infections, contre des facteurs exogènes tels que le brouillard de fumée, la fumée de cigarette et contre l'agression de substances nocives et/ou irritantes, en particulier les tensioactifs et/ou le contact fréquent avec l'eau.

9. Procédé non thérapeutique pour la vitalisation des cheveux, l'excitation du métabolisme énergétique dans les follicules pileux, l'activation de follicules pileux, la stimulation ou le renforcement de la croissance des cheveux, l'épaississement des cheveux, le traitement de la chute des cheveux et l'influence (notamment l'excitation) de la synthèse de la kératine ou la mise en forme des cheveux, **caractérisé en ce que** l'on applique un agent selon l'une quelconque des revendications 1 à 6 sur les cheveux ou la peau poilue.

10. Procédé non thérapeutique pour l'épaississement de cellules et couches de cellules épithéliales, en particulier sur la peau, l'amélioration de la résistance de la peau, le renforcement de l'épiderme, la réduction de l'amincissement de la peau, en particulier la lutte contre les signes du vieillissement de la peau, la réduction de la perte d'eau transépidermique de la peau, l'amélioration de l'hydratation cutanée, la protection de la peau contre les infections, contre des facteurs exogènes tels que le brouillard de fumée, la fumée de cigarette, et contre l'agression de substances nocives et/ou irritantes, en particulier les tensioactifs et/ou le contact fréquent avec l'eau, **caractérisé en ce que** l'on applique un agent selon l'une quelconque des revendications 1 à 6 sur la peau.
